# EUROPÄISCHE PATENTANMELDUNG

(11) **EP 1 864 686 A1**
(43) Veröffentlichungstag der Anmeldung: **12.12.2007**
(21) Anmeldenummer: 06015707.0
(22) Anmeldetag: 27.07.2006
(51) Int. Cl.: A61L 27/30, A61L 27/56

(54) **Verfahren zur Herstellung eines medizinischen Implantats durch Kaltgasspritzen**

(30) Priorität: 01.06.2006 DE 102006025682
(71) Anmelder: Linde Aktiengesellschaft, 80807 München (DE); Pelvis Holding AG, 5622 Waltenschwil (CH)
(72) Erfinder: García Forgas, Jorge, 8424 Gubrach (CH); Heinrich, Peter, 82110 Germering (DE); Kreye, Prof. Dr. Heinrich, 22175 Hamburg (DE); Salito, Armando, 5610 Wohlen (CH)
(74) Vertreter: Gellner, Bernd

(57) **Zusammenfassung**

Bei einem Verfahren zur Herstellung eines medizinischen Implantats mit einer gewebefreundlichen Oberfläche wird das Implantat beschichtet. Die Beschichtung erfolgt erfindungsgemäß durch Kaltgasspritzen mit einem Partikelwerkstoff.

## Beschreibung

Die Erfindung betrifft ein Verfahren zur Herstellung eines medizinischen Implantats und ein medizinisches Implantat. Insbesondere betrifft die Erfindung ein Verfahren zur Herstellung eines medizinischen Implantats mit einer gewebefreundlichen Beschichtung.

In der Medizintechnik ist der Einsatz von Implantaten inzwischen weit verbreitet. Da die Lebenserwartung der Menschen steigt, kommt es z.B. zu vermehrten Ve r-schleißerkrankungen der Gelenke. Hinzu kommt die oftmals höhere Belastung der Gelenke durch das Ausüben von Extremsportarten. Zur Behandlung solcher Erkrankungen bleibt oftmals nur der Einsatz künstlicher Gelenke, die als Implantate in die Knochen eingesetzt werden.

Besonders häufig werden medizinische Implantate bei Endoprothesen, in der Orthopädie, der (Multi)Traumabehandlung und der Zahnmedizin sowie im Bereich der Wirbelsäule eingesetzt.

Die Befestigung der Implantate, die mit einem Knochen fest verb unden werden müssen, erfolgt über zwei bekannte Verfahren. Zum einen durch Einwachsen und zum anderen durch Einzementieren.

Dabei ist es für das Verfahren des Einwachsens notwendig, dass die Oberfläche des Implantats gewebefreundlich ist. Hierfür kann auch nur eine Beschichtung, z. B. aus. Titan, auf das jeweilige Implantat aufgebracht werden, die knochenfreundlich ist. D.h. es wird eine Schicht aufgebracht, die vom Körper nicht abgestoßen wird und das Einwachsen behindert, sondern die durch ihr Material das Einwachsen fördert.

Ein solches Implantat mit einer Oberflächenbeschichtung, die das Einwachsen fördert, ist aus der DE 10 2004 021 739 A1 bekannt, bei der ein medizinisches Implantat eine biokompatible poröse Oberflächenschicht aufweist, die Selen enthält und elektrolytisch aufgebracht wird.

Vermehrt werden auch Implantate aus Kunstoffen wie z.B. Polyethylen hergestellt. Hier bestehen große Schwierigkeiten, eine knochenfreundliche Schicht auf den Kunststoff aufzubringen.

Bekannt sind Verfahren des thermischen Spritzens, um z.B. Metallbeschichtungen auf metallische Werkstoffe aufzubringen. Bei diesen werden Partikel des Beschichtung s-werkstoffs in der Spritzpistole aufgeschmolzen und treffen in flüssigem Zustand oder zumindest noch teilweise geschmolzen auf das Substrat auf. Eingesetzt wird be i-spielsweise das Plasmaspritzen unter umgebender Atmosphäre oder das Plasmaspritzen im Vakuum oder in Schutzgasumgebung.

Nachteile ergeben sich bei der Beschichtung von Implantaten in der Zusammensetzung der Beschichtung und im Schichtaufbau, da es zwangsläufig zu einem Aufschmelzen des Schichtwerkstoffes kommt. Unter anderem kann es zu einem Aufox i-dieren, einer nicht konstanten und somit reproduzierbar herstellbaren Porosität, oder zu nicht gewünschten Verunreinigungen kommen. Aufgrund der Aufoxidation entsteht eine Beschichtung, die problematisch hinsichtlich des Einwachsens ist und der Verträglichkeit von Implantat und Beschichtung ist. Eine unbestimmte Porosität ist von Nachteil, da dies zu einem ungleichmäßigen Einwachsen des Implantats führt. Diese hat zur Folge, dass die Belastung des Implantats nicht gewährleistet ist und Brüche an der Verbindungsstelle des Implantats mit körpereigenem Gewebe nicht hinreichend unterbunden werden. Weiter nachteilig an diesem Stand der Technik ist, dass bei Implantaten aus Kunststoffen der Kunststoff durch die hohen Temperaturen g eschädigt werden kann.

Es ist daher Aufgabe der vorliegenden Erfindung, ein Verfahren zur Herstellung eines medizinischen Implantats zur Verfügung zu stellen, mit dem sich eine gewebefreundliche Oberfläche des Implantats erreichen lässt und welche die vorbeschriebenen Nachteile vermeidet.

Diese Aufgabe wird durch ein Verfahren zur Herstellung eines medizinischen Implantats nach Anspruch 1 und ein medizinisches Implantat nach Anspruch 9 gelöst. Vorteilhafte Weiterbildungen werden durch die Unteransprüche angegeben.

Durch ein Verfahren zur Herstellung eines medizinischen Implantats mit den Merkmalen des Anspruchs 1 ist es möglich, fest und dauerhaft auch bei schwierigen Werkstoffpaarungen eine Beschichtung aus einem gewebefreundlichen, das Einwachsen fördernden Werkstoff auf einem medizinischen Implantat anzubringen.

Beim Kaltgasspritzen wird ein komprimiertes und auf einige hundert C° aufgeheiztes Gas einer Spritzpistole zugeführt und dort in einer Lavaldüse auf Umgebungsdruck entspannt. Dabei sind Drücke und Lavaldüse so ausgelegt, dass das Gas mehrfache Schallgeschwindigkeit erreicht. Der Partikelwerkstoff wird mit einem Trägergas im Bereich des Düseneintritts in den Gasstrom geleitet und mit ihm beschleunigt. Die hohe Gastemperatur bewirkt eine Erhöhung der Strömungsgeschwindigkeit in der Düse und begünstigt die Verformung der Partikel beim Aufprall aufgrund der höheren Partikeltemperatur. Die Gastemperatur liegt aber stets unterhalb der Schmelztemper a-tur des Spritzmaterials, sodass kein Schmelzen der Partikel stattfinden kann. Erst beim Aufprall auf das Substrat steigt durch plastische Verformung unter sehr hohen Dehnraten die Temperatur an den kollidierenden Grenzflächen und führt zu einer Verschweißungen des Partikelwerkstoffs mit dem Substrat sowie untereinander. Dazu muss jedoch eine Mindestaufprallgeschwindigkeit überschritten werden, die so genannte kritische Geschwindigkeit. Der Mechanismus und die Qualität der Verschweißung ist mit dem Explosivschweißen vergleichbar und ermöglicht auch eine Beschichtung bei schwierigen Werkstoffpaarungen.

Durch das erfindungsgemäße Aufbringen der Beschichtung mittels Kaltgasspritzen wird es möglich, eine nahezu oxidfreie Beschichtung auf das Implantat aufzubringen und zugleich ist die Porosität bei der Schichtaufbringung sehr gut steuerbar. Dadurch werden Unverträglichkeiten mit dem körpereigenem Gewebe vermieden und ein gleichmäßiges Einwachsen des Implantats wird gewährleistet. Durch eine geeignete Wahl für den Wert der Porosität wird zudem das Anwachsen des Implantats und die Festigkeit des Implantats nach dem Anwachsen unterstützt.

In günstiger Ausführungsform ist der Partikelwerkstoff Titan. Auch andere gewebeverträgliche Werkstoffe können verwendet werden.

Titan ist gut verträglich und führt zu keinen Abstoßungsreaktionen und ist daher besonders geeignet als Beschichtungswerkstoff.

Die Partikelgröße kann bis zu 300 µm betragen. Meist werden jedoch Partikel mit einer Größe von 1 bis 100 µm, vorzugsweise von 1 bis 50 µm verwendet.

In günstiger Ausführungsform ist die Rauhigkeit größer als 100 Rz.

Dadurch kann das umgebende Gewebe besser und fester mit dem Implantat ve rwachsen. Insbesondere durch die Wahl der Partikelgröße und der Parameter, unter denen das Kaltgasspritzen durchgeführt wird, lässt sich die Rauhigkeit oder Rautiefe gut steuern.

Vorteilhaft beträgt die Porosität der Beschichtung 20 bis 40%.

Durch einen Anteil von 20 bis 40% von Hohlräumen am Volumen der Beschichtung kann das umgebende Gewebe gut in die Beschichtung hineinwachsen und so mit dem Implantat verwachsen.

Vorteilhaft kann die zu beschichtende Implantatoberfläche aus einem Kunststoff b e-stehen und in einem ersten Schritt können Partikel so auf den Kunststoff gespritzt werden, dass die Partikel in den Kunststoff eindringen. Der Kunststoff kann dadurch die Partikel teilweise umschließen, so dass nur ein Teil des Partikels noch aus dem Kunststoff hervorsteht und in einem zweiten Schritt wird auf die pigmentartig über die Oberfläche des Implantats verteilt angeordneten Partikel eine umfassende Beschichtung durch Kaltgasspritzen aufgebracht. Vorteilhafter wird auch der erste Schritt der Beschichtung mittels Kaltgasspritzen ausgeführt, da dann die Beschichtung in einem Arbeitsschritt erfolgen kann, bei welchem lediglich die Spritzparameter verändert we r-den brauchen. Jedoch ist es auch möglich den ersten Schritt mit einem anderen Verfahren aufzubringen.

Vorteilhaft ist der Kunststoff Polyethylen. Jedoch können auch andere Kunststoffe beschichtet werden.

In günstiger Ausführungsform erfolgt das Kaltgasspritzen mit Stickstoff, Helium oder einem Gemisch aus diesen beiden. In manchen Fällen ist es auch möglich, mit Luft zu spritzen.

Die Aufgabe wird auch durch ein medizinisches Implantat gelöst, das nach einem der zuvor geschilderten Verfahren hergestellt wurde.

Beispielsweise kann eine Beschichtung eines medizinischen Implantats aus Metallwerkstoff mit reinem Titan erfolgen, das als Partikel mit einer Größe von 5 µm bis zu 300 µm verspritzt wird. Dabei wird eine Beschichtung mit einer Schichtstärke von 125 - 200 aufgebracht. Die Porosität der Beschichtung beträgt zwischen 20 und 40% und die gemittelte Rautiefe Rz mehr als 100. Die Haftzugfestigkeit der Beschichtung auf dem Substrat kann mehr als 40 N/mm² betragen.

Durch die Rauhigkeit, die Porosität und die gute Gewebeverträglichkeit des Titans wird ein Einwachsen des Implantats in das Gewebe gefördert. Insbesondere kann Gewebe in die Höhlräume einwachsen und sich mit der rauen Oberfläche verbinden. Durch das Kaltgasspritzen ist eine Beschichtung auch bei ungünstigen Werkstoffkombinationen möglich.

In einem alternativen Beispiel erfolgt eine Beschichtung eines medizinischen Implan-tats aus Polyethylen. Titan wird als Partikel mit einer Größe von 5 µm bis zu 300 µm verspritzt. Dabei wird eine Beschichtung mit nicht festgelegter Schichtdicke, nicht festgelegter Porosität der entstehenden Schicht sowie einer gemittelten Rautiefe Rz von mehr als 100 erzeugt, die gut gewebeverträglich ist und ein Einwachsen des Im p-lantats in das Gewebe ermöglicht. Auch hier kann die Haftzugfestigkeit der Beschichtung auf dem Substrat mehr als 40 N/mm² betragen. Zunächst werden Partikel auf das Polyethylen gespritzt, wobei die Partikel durch ihre kinetische Energie in die Oberfläche des Polyethylen eindringen und von diesem fest umschlossen werden bis auf einen über die Oberfläche vorstehenden Bereich. Dadurch ergeben sich pigmentartig auf der Oberfläche angeordneten- Partikel, die als Substrat für die Beschichtung durch das weitere Kaltgasspritzen dienen. Dabei können die Eindringtiefe und Festigkeit, mit der die Partikel in das Polyethylen eingebettet werden, durch die Temperatur und Geschwindigkeit der Partikel gesteuert werden.

## Patentansprüche

1. Verfahren zur Herstellung eines medizinischen Implantats mit einer gewebefreun dlichen Oberfläche,
bei dem das Implantat beschichtet wird,
**dadurch gekennzeichnet,**
**dass** die Beschichtung durch Kaltgasspritzen mit einem Partikelwerkstoff erfolgt.

2. Verfahren nach Anspruch 1,
**dadurch gekennzeichnet,**
**dass** der Partikelwerkstoff aus Titan besteht.

3. Verfahren nach Anspruch 1 oder 2,
**dadurch gekennzeichnet,**
**dass** die Partikelgröße bis zu 300 µm beträgt.

4. Verfahren nach einem der vorhergehenden Ansprüche,
**dadurch gekennzeichnet,**
**dass** die Beschichtung mit einer gemittelten Rautiefe Rz größer als 100 hergestellt wird.

5. Verfahren nach einem der vorhergehenden Ansprüche,
**dadurch gekennzeichnet,**
**dass** die Beschichtung mit einer Porosität von 20- 40% hergestellt wird.

6. Verfahren nach einem der vorhergehenden Ansprüche,
**dadurch gekennzeichnet,**
**dass** die zu beschichtende Implantatoberfläche aus einem Kunststoff besteht und in einem ersten Schritt Partikel so auf den Kunststoff gespritzt werden, dass die Partikel in den Kunststoff eindringen, der Kunststoff die Partikel teilweise umschließt und auf die pigmentartig auf der Oberfläche angeordneten Partikel als Substrat die Beschichtung durch Kaltgasspritzen erfolgt.

7. Verfahren nach Anspruch 6,
**dadurch gekennzeichnet,**
**dass** der Kunststoff Polyethylen ist.

8. Verfahren nach einem der vorhergehenden Ansprüche,
**dadurch gekennzeichnet,**
**dass** das Kaltgasspritzen mit Stickstoff, oder Helium oder einem Gemisch aus diesen erfolgt.

9. Medizinisches Implantat mit einer Beschichtung,
**dadurch gekennzeichnet,**
**dass** die Beschichtung nach einem Verfahren nach einem der Ansprüche 1 bis 8 hergestellt ist.
